# EUROPÄISCHE PATENTSCHRIFT

(11) **EP 2 480 264 B1**
(45) Veröffentlichungstag und Bekanntmachung des Hinweises auf die Patenterteilung: **05.09.2018**
(21) Anmeldenummer: 10760580.0
(22) Anmeldetag: 17.09.2010
(51) Int. Cl.: A61M 1/06, A61M 1/00

(54) **VORRICHTUNG UND VERFAHREN ZUM ABPUMPEN VON MENSCHLICHER MUTTERMILCH**
DEVICE AND METHOD FOR EXPRESSING HUMAN BREAST MILK
DISPOSITIF ET PROCÉDÉ DESTINÉS À POMPER LE LAIT MATERNEL HUMAIN

(30) Priorität: 22.09.2009 US 244636 P
(43) Veröffentlichungstag der Anmeldung: 01.08.2012
(73) Patentinhaber: Medela Holding AG, 6340 Baar (CH)
(72) Erfinder: WEBER, Beda, CH-5643 Sins (CH); FURRER, Etienne, CH-6300 Zug (CH); SCHLIENGER, André, CH-8933 Maschwanden (CH); SILVER, Brian, H., Cary IL 60013 (US); WÄCKERLIN, Daniela, CH-6340 Baar (CH); FELBER, Armin, CH-6003 Luzern (CH)
(74) Vertreter: Clerc, Natalia
(86) Internationale Anmeldenummer: PCT/CH2010/000225
(87) Internationale Veröffentlichungsnummer: WO 2011/035447

(56) Entgegenhaltungen:
- EP-A1- 1 219 833
- EP-A2- 2 127 690
- WO-A2-2008/057218
- US-A1- 2004 087 898
- US-A1- 2005 043 677
- US-A1- 2005 222 536
- US-A1- 2007 078 383
- US-A1- 2008 090 445

## Beschreibung

### TECHNISCHES GEBIET

Die vorliegende Erfindung betrifft eine Vorrichtung zum Abpumpen von menschlicher Muttermilch gemäss Oberbegriff des Patentanspruchs 1, sowie ein Verfahren zum Betreiben einer Vorrichtung zum Abpumpen von menschlicher Muttermilch gemäss Oberbegriff des Patentanspruchs 13.

### STAND DER TECHNIK

Vorrichtungen zum Abpumpen von menschlicher Muttermilch sind hinlänglich bekannt. Grundsätzlich gibt es zwei verschiedene Typen: die ersten werden manuell bedient, d.h. der für das Abpumpen notwendige Unterdruck wird durch manuelle Betätigung der Vakuumpumpe erzeugt. Bei den zweiten Typen ist die Vakuumpumpe elektrisch betrieben, wobei die Vakuumpumpe ans Stromversorgungsnetz angeschlossen sein kann und/oder über eine Batterie bzw. einen anderen Energiespeicher betrieben werden kann. Beispiele hierfür sind WO 96/22116, US 2009/0099511, US 2008/0287037, US 7 094 217 und US 2008/0039781.
Diese Vakuumpumpen sind entweder direkt oder über Vakuumleitungen mit einer Brusthaube verbunden. Die Brusthaube weist üblicherweise einen trichterförmigen Teil auf zur Aufnahme eines Teils der Mutterbrust inklusive der Brustwarze. In der Regel geht dieser trichterförmige Teil in einen hohlzylinderförmigen Teil über, an welchem einerseits entweder direkt die Vakuumpumpe oder die Saugleitung angeschlossen ist und welcher andererseits ebenfalls direkt oder über eine Milchleitung mit einem Milchsammelbehälter verbunden ist. Es ist bekannt, Brusthauben entsprechend der Grösse der Brust auszuwählen. Es sind insbesondere Brusthaubensets bekannt, bei welchen die Grösse des trichterförmigen Teils der Mutter entsprechend gewählt werden kann.

Es sind im Stand der Technik auch relativ kleine Brusthauben bekannt. So offenbart US 6 379 327 eine tragbare, sogenannte "hands-free" Abpumpvorrichtung. "hands-free" bedeutet in diesem Zusammenhang, dass die gesamte Vorrichtung nach dem Einschalten ohne Hände funktioniert, d.h. dass weder die Pumpe noch die Brusthaube von Hand gehalten werden müssen. Bei US'327 ist hierfür eine kleine trichterförmige Brusthaube mit Bändern an der Brust befestigt. Ein erster Schlauch führt von der Brusthaube zu einer Vakuumpumpe, welche in einem Gürtel gehalten ist. Ein zweiter Schlauch führt von der Brusthaube in einen Milchsammelbehälter, welcher im gleichen Gürtel getragen werden kann.

Auch WO 02/102437 und WO 2008/137678 zeigen "hands-free" Abpumpvorrichtungen. Hier ist die Brusthaube jeweils in einem Pumpengehäuse integriert und dient gleichzeitig als Membran zur Erzeugung eines Unterdrucks.

US 949 414 beschreibt eine trichterförmige Brusthaube, welche unter einem Büstenhalter angeordnet werden kann. Ein Vakuum wird nicht angelegt, sondern ein Schlauch führt von der Brusthaube zu einem Baby, welches durch Saugen am Schlauch die gewünschte Milch erhalten soll.

US 6 440 100 zeigt eine Vorrichtung zum Abpumpen von Muttermilch mit einer kleinen, unter dem Büstenhalter tragbaren Brusthaube. Ein Milchschlauch führt von der Brusthaube zu einem Milchsammelbehälter. Dieser ist über einen Vakuumschlauch mit einer Vakuumquelle verbunden. Der Milchsammelbehälter wird mittels der Vakuumquelle evakuiert, wobei der Unterdruck über den Milchschlauch an die Brusthaube angelegt wird. Dank dem im Milchsammelbehälter herrschenden Unterdruck soll nun abgepumpte Milch über den Milchschlauch in den Milchsammelbehälter gelangen. Alternativ kann der Milchsammelbehälter selber als Vakuumpumpe dienen. Diese Vorrichtung weist den Nachteil auf, dass das relativ grosse Volumen des Milchsammelbehälters ebenfalls evakuiert werden muss. Dieses sogenannte Totvolumen schränkt die Leistungsfähigkeit der Vorrichtung massiv ein.

US 2004/087898 und WO 2008/057218 offenbaren Brusthauben mit einer Medientrennmembran.
US 2007/078383 offenbart eine handbetriebene Brustpumpe mit einer Vakuummembran. EP 1 219 833 offenbart eine Brustpumpe mit einer Pumpmembran, welche einen Sauganschluss und einen Auspuff aufweist.

### DARSTELLUNG DER ERFINDUNG

Es ist eine Aufgabe der Erfindung, eine verbesserte Vorrichtung und ein verbessertes Verfahren zum Abpumpen von menschlicher Muttermilch zu schaffen.

Diese Aufgabe lösen eine Vorrichtung mit den Merkmalen des Anspruchs 1, sowie ein Verfahren mit den Merkmalen des Anspruchs 13. Die erfindungsgemässe Vorrichtung zum Abpumpen von menschlicher Muttermilch weist eine Brusthaube zur Anlage an eine Mutterbrust, eine Vakuumpumpe zur Erzeugung eines Vakuums, eine die Brusthaube mit der Pumpkammer verbindende Leitung zum Übermitteln des erzeugten Vakuums an die Brusthaube und eine Kammer auf. Die Leitung endet pumpenseitig in einem ersten Anschluss dieser Kammer. Erfindungsgemäss weist die Kammer einen zweiten Anschluss zur Verbindung mit einem Milchsammelbehälter auf. Die zwei Anschlüsse stehen in der Kammer in fluidkommunizierender Verbindung miteinander. Die Leitung bildet während des Abpumpens eine Milchleitung zum Transport von in der Brusthaube abgepumpter Muttermilch zur Kammer. Die Milch wird anschliessend von der Kammer zum Milchsammelbehälter geleitet.
Im erfindungsgemässen Verfahren zum Betreiben einer Vorrichtung zum Abpumpen von menschlicher Muttermilch wird mittels einer Vakuumpumpe ein Vakuum in einer Brusthaube erzeugt, wobei das erzeugte Vakuum von einer Kammer über eine Leitung zur Brusthaube geleitet wird und wobei abgepumpte Milch über die Brusthaube in einem Milchsammelbehälter gesammelt wird. Erfindungsgemäss leitet dieselbe Leitung, welche für die Übermittlung des Vakuums verwendet wird, während dem Abpumpen die in der Brusthaube abgepumpte Milch zur Kammer, wobei die Milch während dem Abpumpen von der Kammer in einen Milchsammelbehälter weitergeleitet wird.
Diese Vorrichtung wechselt somit von einem anfänglichen pneumatischen Pumpsystem zu einem hydraulischen Pumpsystem, wobei dieselbe Leitung verwendet wird. Die abgepumpte Milch agiert dabei als Arbeitsfluid, um weitere Milch aus der Brust abzupumpen. Ferner wärmt diese bereits abgepumpte Milch die Brusthaube, was für die Mutter angenehm ist.

Die erfindungsgemässe Vorrichtung und das erfindungsgemässe Verfahren benötigen nur eine einzige Schlauchverbindung, auch wenn der Milchsammelbehälter nicht direkt an der Brusthaube angeordnet ist. Diese Vorrichtung lässt sich somit auch als "hands-free" Vorrichtung ausgestalten und verwenden. Die Brusthaube kann klein ausgeführt werden und in einem Büstenhalter fixiert werden. Die einzige Leitung lässt sich diskret in der Kleidung verstecken.

Da das gesamte System von Milch geflutet ist und somit keine Vakuumleitung im klassischen Sinne mehr vorhanden ist, ist eine geringere Pumpleistung notwendig, um Muttermilch abzupumpen. Typische Werte für die Luftförderleistung liegen bei maximal 500 ml/min und für die Milchförderleistung bei maximal 100 ml/min. Die Vakuumpumpe kann somit kleiner und leichter ausgestaltet werden, was wiederum für Aussenstehende weniger auffällig ist. Die Mutter kann diese Vakuumpumpe diskreter verwenden. Dank der geringeren Anforderung an die Pumpleistung ist die Vakuumpumpe im Gebrauch zudem leiser, was wiederum den Komfort und die Diskretion erhöht.

Da das gesamte System, d.h. das gesamte System bis auf den pumpaggregatseitigen oder antriebseitigen Bereich der Vakuumpumpe, mit Milch geflutet ist und keine oder nur sehr kleine luftgefüllten Toträume vorhanden sind, lässt sich das angelegte Vakuum leichter kontrollieren. Der an der Brusthaube anliegende Unterdruck entspricht auch eher dem in der Vakuumpumpe erzeugten Vakuum.

Diese Milchleitung lässt sich auf verschiedene Weisen verwirklichen. In einer bevorzugten Ausführungsform ist eine Scheidewand vorhanden ist, welche einen Antrieb der Vakuumpumpe und die Leitung voneinander trennt. Die Kammer wird dadurch von der Scheidewand in einen brusthaubenseitigen Bereich und einem antriebsseitigen Bereich unterteilt. Die zwei Bereiche sind vollständig voneinander getrennt und nur über die Membran miteinander verbunden. Somit ist gewährleistet, dass weder Milch in den antriebsseitigen Bereich der Vakuumpumpe gelangt, noch dass Verschmutzungen oder Luft des antriebsseitigen Bereichs in die milchführende Leitung und somit in die Brusthaube und den Milchsammelbehälter gelangt. Vorzugsweise ist diese Scheidewand eine Membran.
Diese Membran ist in einer bevorzugten Ausführungsform angetrieben und dient zur Förderung der abgepumpten Milch. Dadurch lässt sich die Milch unabhängig von der relativen Lage der Brusthaube, des Milchsammelbehälters und der Vakuumpumpe zueinander abpumpen. Die Mutter kann beispielsweise auch liegend abpumpen. Dies ist insbesondere bei einer "hands-free" Ausführung optimal, da sich die Mutter auch bücken und im Allgemeinen sehr frei bewegen kann.
Es lassen sich die unterschiedlichsten Vakuumpumpen mit der erfindungsgemässen vakuumübermittelnden und milchführenden Leitung verwenden. Vorzugsweise, jedoch nicht zwingend, wird jeweils eine einzige Membran sowohl für den Transport der Milch wie auch für die Medientrennung eingesetzt.
Vorzugsweise ist die Vakuumpumpe eine Membranpumpe, wobei die Kammer die vakuumerzeugende Pumpkammer der Vakuumpumpe ist und die Membran die für die Erzeugung des Vakuums verwendete Membran der Pumpkammer ist.

Eine bevorzugte Vakuumpumpe zum Abpumpen von menschlicher Muttermilch mittels einer Brusthaube weist einen Antrieb und eine vom Antrieb zyklisch antreibbare Membran auf, wobei die Membran in einer Kammer angeordnet ist, wobei die Membran die Kammer in einen antriebsseitigen Teil und einen brusthaubenseitigen Teil trennt und wobei der brusthaubenseitige Teil einen Vakuumanschluss zur Erstellung einer Verbindung zur Brusthaube aufweist. Der brusthaubenseitige Teil der Kammer weist ferner einen Milchanschluss zur Erstellung einer Verbindung mit einem Milchsammelbehälter auf und der Vakuumanschluss und der Milchanschluss stehen über den brusthaubenseitigen Teil der Kammer in fluidkommunizierender Verbindung miteinander. Vorzugsweise ist diese Vakuumpumpe eine Membranpumpe und die Kammer ist die vakuumerzeugende Pumpkammer.
Diese Vakuumpumpe kann, muss jedoch nicht die oben genannte vakuumübermittelnde und milchführende Leitung aufweisen. Es ist auch möglich, dass bei dieser Vakuumpumpe die Brusthaube direkt an den ersten Anschluss der Kammer angeschlossen ist.

In einer bevorzugten Ausführungsform ist die Vakuumpumpe eine elektrische Membranpumpe. Die Membran der Membranpumpe bildet dabei vorzugsweise die als Milchantrieb und Scheidewand dienende oben genannte Membran. Der Antrieb der Membran dient vorzugweise gleichzeitig zur Erzeugung des Vakuums in der Pumpkammer und zur Förderung des Milchflusses. Dank der Dreifachfunktion der Membran lässt sich das Vakuum besser kontrollieren.

Vorzugsweise weist die Membran einen im Wesentlichen kreisförmigen Grundriss auf. Vorzugsweise ist die Membran in ihrem mittleren Bereich, vorzugsweise ihrer Mitte, angetrieben.

Es lassen sich auch andere Arten von Membranpumpen verwenden, welche beispielsweise eine Flachspule oder eine Tauchspule als Antriebssystem aufweisen. Auch alternative Pumpen ohne Membran können eingesetzt werden. Des weiteren können manuell betriebene Pumpen verwendet werden.

Vorzugsweise ist ein Rückschlagventil oder ein anderes Einwegventil vorhanden, welches eine Verbindung zwischen der Kammer und dem Milchsammelbehälter schafft. Dadurch wird die Verbindung zum Milchsammelbehälter erst geöffnet, wenn das restliche System bereits möglichst vollständig mit Milch gefüllt ist bzw. wenn ein genügend grosser Druck in der Kammer vorliegt. Auch hier wird wieder eine hohe Belastung der Vakuumpumpe durch allfällige luftgefüllte Totvolumina vermieden. Der zweite Anschluss und somit auch das Ventil sind in bestimmungsgemässer Gebrauchslage der Vakuumpumpe unten angeordnet. Es befindet sich im brusthaubenseitigen, auch leitungsseitigen genannten Bereich der Kammer. Der erste Anschluss ist hingegen vorzugsweise im oberen Bereich der Kammer angeordnet. Dadurch wird Luft der Kammer in den Milchsammelbehälter geleitet und luftgefüllte Totvolumina bzw. Luftblasen im System werden vermieden.

Die erfindungsgemässe Vorrichtung und das erfindungsgemässe Verfahren lassen sich mit allen Arten von Brusthauben verwenden. Vorzugsweise werden jedoch kleine Brusthauben verwendet, um auch hier das Totvolumen möglichst klein zu halten.

Vorzugsweise wird eine Brusthaube verwendet, welche lediglich die Brustwarze und maximal die Areola umgibt. Diese lässt sich nicht nur einfach in einem "hands-free" System einsetzen, z.B. in einem Büstenhalter halten, sondern sie weist aufgrund ihrer geringen Grösse kaum luftgefüllte Bereiche auf. Dies wirkt sich positiv auf die Vakuumpumpe aus, da diese weniger Leistung benötigt und somit geräuscharmer arbeiten kann. Des Weiteren lässt sie sich auch aus diesem Grund kleiner ausbilden.

Derartige Brusthauben weisen zudem den Vorteil auf, dass weniger Gewebebewegung der Brust innerhalb der Haube stattfinden kann. Die Brusthauben können dadurch dichter am Gewebe anliegen. Dadurch ist eine geringere Pumpleistung notwendig. Die Pumpe kann wiederum kleiner ausgebildet werden und arbeitet geräuscharmer.

Erfindungsgemäss weist die Vorrichtung eine Brusthaube auf, welche sich optimal an die Form der menschlichen Mutterbrust anpasst und somit je nach Wunsch eine dichte oder eine weniger dichte Verbindung mit der Brust schafft.

Eine derartige erfindungsgemässe Brusthaube weist einen rohrförmigen Stutzen und einen daran einstückig angeformten Trichter zur Auflage an eine Mutterbrust auf, wobei der Trichter sich zu seiner freien, dem Stutzen abgewandten Seite hin erweitert und wobei ein Kanal vorhanden ist, welcher sich von einem brustseitigen Ende des Trichters durchgehend bis zu einem, diesem Ende gegenüberliegenden, pumpenseitigen Ende des Stutzens erstreckt und welcher zum Anlegen eines Vakuums an die Mutterbrust und zum Abfliessen der abgepumpten Muttermilch dient. Erfindungsgemäss ist der Trichter flexibler ausgebildet als der Stutzen. Der Trichter weist einen sich über einen wesentlichen Teil seiner Länge erstreckenden Hauptbereich mit einem ersten Öffnungswinkel des Kanals und einen brustseitigen Endbereich mit einem zweiten Öffnungswinkel des Kanals auf. Der erste Öffnungswinkel ist bei Nichtgebrauch kleiner als der zweite Öffnungswinkel, wobei im Gebrauchszustand mindestens der erste Öffnungswinkel durch axialen Druck auf die Brusthaube vergrösserbar ist.

Diese Brusthaube lässt sich auch in anderen Vorrichtungen zum Abpumpen von Muttermilch einsetzen und ist nicht auf die Verwendung von gleichzeitig vakuumübertragenden und milchführenden Leitungen oder von den oben beschriebenen Kammern beschränkt.

Im Gegensatz zu den bekannten Brusthauben und zum natürlichen Saugen eines Baby wird die Brustwarze in einer derartigen Brusthaube üblicherweise nicht auf das 2.5 fache ihrer Länge gedehnt. Dies ist für die Mutter, insbesondere bei schmerzenden Brustwarzen, angenehm.

Bei Nichtgebrauch sind typische Werte des ersten Öffnungswinkels der Brusthaube ≤ 5° (kleiner oder gleich 5°) und des zweiten Öffnungswinkels 90° bis 160°. Im Gebrauch und je nach angelegtem axialen Druck lässt sich zumindest der zweite Öffnungswinkel bis zu 120°, vorzugsweise bis zu 160° vergrössern.

Vorzugsweise weist der Trichter einen brustseitigen Durchmesser von 5 mm bis 40 mm und eine Länge von 10 mm bis 40 mm auf, so dass im Gebrauchszustand die Brustwarze und maximal die Areola von der Brusthaube umfasst ist. Milchkanäle im Brustgewebe sind vorzugsweise nicht von der Brusthaube umgeben.

Vorzugsweise ist zwischen Stutzen und Trichter ein Übergangsbereich vorhanden mit einem dritten Öffnungswinkel des Kanals, wobei der dritte Öffnungswinkel bei Nichtgebrauch grösser ist als der erste Öffnungswinkel.

Typische Werte des dritten Öffnungswinkels sind 60° bis 150°.

Vorzugsweise schliesst der Hauptbereich unmittelbar an den Übergangsbereich an. Auch der Endbereich schliesst vorzugweise unmittelbar an den Hauptbereich an.

In einer bevorzugten Ausführungsform weist der Stutzen eine um ein Vielfaches grössere Wandstärke auf als der Trichter. Zusätzlich oder alternativ kann der Stutzen auch aus einem Material mit grösserer Shore Härte gebildet sein.

Vorzugsweise ist im Übergangsbereich zwischen Stutzen und Trichter ein äusserer Anschlag vorhanden, welcher dem äusseren Umfang des Stutzens vorsteht.

Der Stutzen lässt sich gut in eine Aufnahme einschieben, wenn er in seinem äusseren Umfang konisch zum Trichter hin erweiternd ausgebildet ist.

Diese erfindungsgemässe Brusthaube ist vorzugsweise aus Silikon gefertigt und weist vorzugsweise eine Shore A Härte von 30 bis 70 auf. Vorzugsweise weist der Trichter eine Shore A Härte von circa 50 auf. Der Stutzen weist vorzugsweise eine Shore A Härte von circa 70 auf.

Damit diese relativ kleine und kompakte Brusthaube gut in der Hand gehalten werden kann, ist sie vorzugsweise in einem Brusthaubenset mit einem Kopplungsteil angeboten. Das Kopplungsteil dient zur dichtenden Aufnahme des Stutzens der Brusthaube, wobei es zylinderförmig ausgebildet ist und auf einer Seite mit einem Boden verschlossen ist, so dass ein Sackloch zur Aufnahme des Stutzens gebildet ist. Es ist eine Anschlussöffnung vorhanden, welche in fluidkommunizierender Verbindung mit dem Kanal der Brusthaube steht.

Im zusammengebauten Zustand endet ein brusthaubenfernes, stirnseitiges Ende des Stutzens vorzugsweise beabstandet zum Boden des Kopplungsteils. Vorzugsweise ist die Anschlussöffnung azentrisch im Kopplungsteil angeordnet.

Ist die Anschlussöffnung in einem oberen Bereich des Kopplungsteils angeordnet und das Kopplungsteil weist eine Markierung auf, welche "oben" im Raum definiert, so werden beim Abpumpen stehende Luftblasen im Kopplungsteil vermieden und luftgefüllte Toträume eliminiert.

Um den konischen Stutzen einfacher einführen zu können, verjüngt sich auch das Sackloch des Kopplungsteils im Durchmesser zum Boden hin.

Vorzugsweise ist das Kopplungsteil steif ausgebildet, was die Einführung des Stutzens erleichtert und die Stabilität erhöht. Das Teil lässt sich somit einfacher halten oder befestigen.

Die Kombination der drei Elemente
- vakuumübermittelnde und milchführende Leitung
- Membran der Pumpkammer mit ihrer Dreifachfunktion
- die klein ausgebildete, Totvolumen vermeidende Brusthaube
führt zu einer Vorrichtung, welche äusserst klein und geräuscharm ausgebildet werden kann und ferner optimal für jegliche Art von Anwendung, insbesondere einer "hands-free" Anwendung, geeignet ist.

Weitere vorteilhafte Ausführungsformen und Varianten des Verfahrens sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine Explosionsdarstellung einer erfindungsgemässen Vorrichtung in einer ersten Ausführungsform, wobei eine Seitenwand der Vakuumpumpe nicht dargestellt ist;
- Figur 2: die Vorrichtung gemäss Figur 1 in gebrauchsmässigem Zusammenbau, wobei eine Seitenwand der Vakuumpumpe nicht dargestellt ist;
- Figur 3: eine Explosionsdarstellung der Vakuumpumpe von einer ersten Seite;
- Figur 4: die Vakuumpumpe gemäss Figur 3 von einer zweiten Seite;
- Figur 5: die Vakuumpumpe gemäss Figur 3 im gebrauchsmässigen Zusammenbau, jedoch ohne Seitenwand;
- Figur 6: eine erfindungsgemässe Vorrichtung in einer zweiten Ausführungsform, wobei eine Seitenwand der Vakuumpumpe nicht dargestellt ist;
- Figur 7a: einen Längsschnitt durch eine erfindungsgemässe Brusthaube, angelegt an eine menschliche Brust mit grösserem Anpressdruck;
- Figur 7b: einen Längsschnitt durch die erfindungsgemässe Brusthaube gemäss Figur 7a, angelegt an eine menschliche Brust mit kleinerem Anpressdruck;
- Figur 8: einen Längsschnitt durch die Brusthaube gemäss Figur 7b mit einem Kopplungsteil in einer ersten Ausführungsform;
- Figur 9: einen Längsschnitt durch ein Kopplungsteil in einer zweiten Ausführungsform;
- Figur 10: einen Längsschnitt durch ein Kopplungsteil in einer dritten Ausführungsform;
- Figur 11: einen Längsschnitt durch das Kopplungsteil gemäss Figur 8;
- Figur 12: eine Explosionsdarstellung eines Teil der erfindungsgemässen Vorrichtung mit einem Pumpaggregat in einer dritten Ausführungsform;
- Figur 13: einen Längsschnitt durch einen Teil der Vorrichtung gemäss Figur 12;
- Figur 14: einen Längsschnitt durch das Pumpaggregat gemäss Figur 12 in einer ersten Ausführungsform und
- Figur 15: einen Längsschnitt durch ein Pumpaggregat in einer zweiten Ausführungsform.

Gleiche Teile sind in den Figuren mit denselben Bezugszeichen versehen.

### BESCHREIBUNG BEVORZUGTER AUSFÜHRUNGSFORMEN

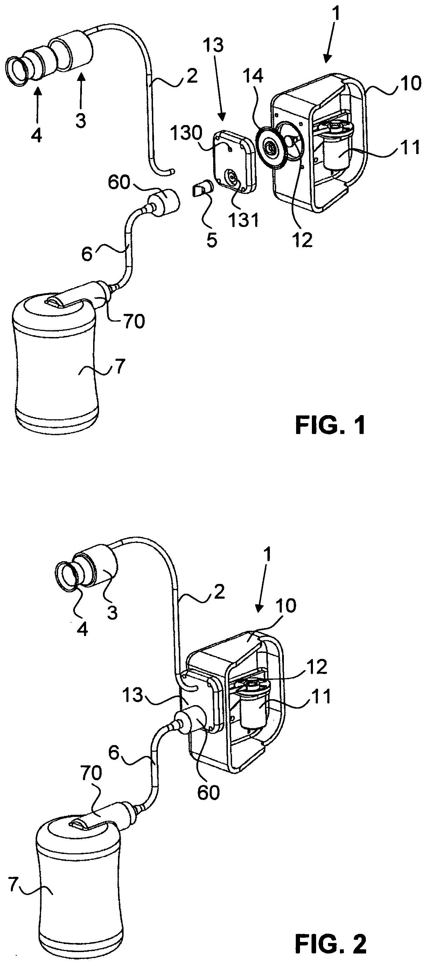

In den Figuren 1 bis 5 ist eine erste Ausführungsform der erfindungsgemässen Vorrichtung dargestellt. Sie weist eine Vakuumpumpe 1, eine erste Leitung 2, ein Kopplungsteil 3, eine Brusthaube 4, ein Rückschlagventil 5, eine zweite Leitung 6 und einen Milchsammelbehälter 7 auf.

Die Brusthaube 4 ist über das Kopplungsteil 3 und der ersten flexiblen Leitung 2 mit der Vakuumpumpe 1 verbunden. Von der Vakuumpumpe 1 führt die zweite flexible Leitung 6 zum Milchsammelbehälter 7, wobei diese Verbindung mit dem Rückschlagventil 5 versehen ist. Die zwei flexiblen Leitungen 2, 6 sind vorzugsweise Schläuche, insbesondere aus Silikon.

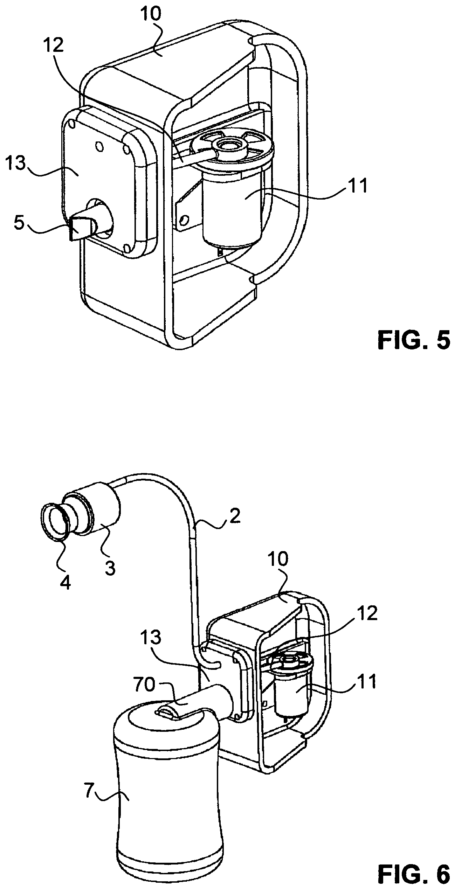

Wie in Figur 6 dargestellt ist, kann alternativ der Milchsammelbehälter 7 auch direkt an der Vakuumpumpe 1 befestigt sein. Hierfür ist vorzugsweise ein geeignet geformter Verbindungsstutzen 70 am Milchsammelbehälter 7 vorhanden, welcher mit einem Gehäuse 10 der Vakuumpumpe lösbar verbindbar ist.

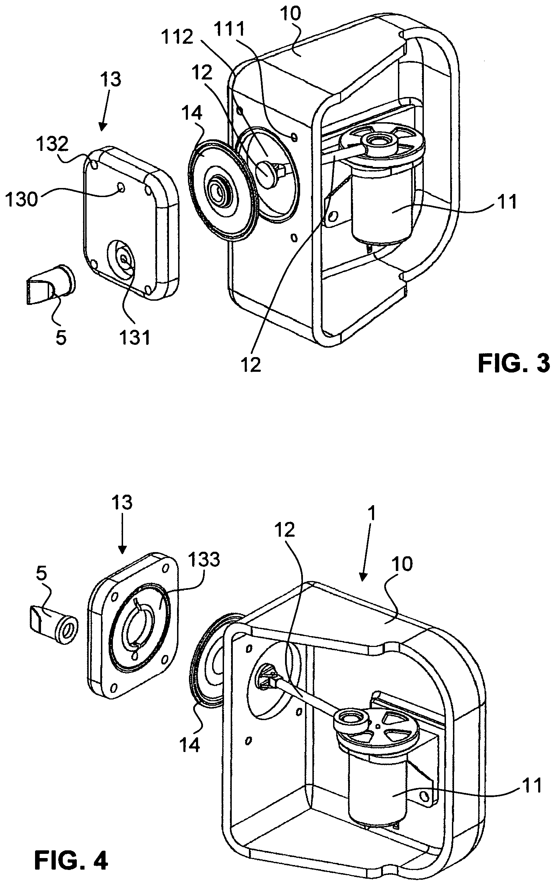

Die Vakuumpumpe 1 weist das genannte Gehäuse 10 auf, wobei in den Zeichnungen eine Seitenwand des Gehäuses 10 nicht dargestellt ist. Dadurch lässt sich das Innere des Gehäuses 10 erkennen. Dieses ist in den Figuren 3 bis 5 gut erkennbar. Im Gehäuse 10 ist ein Elektromotor 11 vorhanden. Er kann netzwerkbetrieben und/oder batteriebetrieben sein.

Eine Kraftübertragungseinheit 12, hier eine mit dem Motor verbundene Pleuelstange, überträgt die Drehbewegung des Motors in eine lineare Bewegung. Die Pleuelstange 12 ist an ihrem zweiten Ende mit einer Membran 14 verbunden. Die Membran 14 ist in einer Ausnehmung 112 des Gehäuses 10, welche ein Teil einer Pumpkammer bildet, angeordnet. Ein mit dem Gehäuse 10 lösbar verbindbarer Deckel 13 hält die Membran 14 in ihrer Position fest.

Anstelle dieses Antriebs lassen sich auch andere Arten von Antrieben, insbesondere auch manuelle Antriebe, einsetzen.

Der Deckel 13 ist vorzugsweise am Gehäuse 10 angeschraubt. Andere Verbindungsarten, sind ebenfalls möglich. Der Deckel 13 weist ebenfalls eine Ausnehmung 133 auf, so dass er einen zweiten Teil der Pumpkammer bildet. Die zwei Teile der Pumpkammer sind durch die Membran 14 voneinander getrennt. Der Deckel 13 kann einstückig oder mehrstückig ausgebildet sein.

Die Wirkungsweise der Membranpumpe wird hier nicht im Detail beschrieben, da sie hinlänglich bekannt ist. Mittels eines Antriebs, hier mittels Motor 11 und Pleuelstange 12, wird die Membran zyklisch hin- und her bewegt, so dass im brusthaubenseitigen oder deckelseitigen Teil der Pumpkammer ein Unterdruck erzeugt wird. Anstelle des hier dargestellten Antriebs lassen sich auch andere Arten von Antrieben verwenden, welche geeignet sind, die Membran 14 zyklisch zu bewegen. Die für die Betreibung der Pumpe notwendige Elektronik sowie die Bedienungselemente sind hier nicht dargestellt. Es können bekannte Mittel verwendet werden. Die Pumpe lässt sich mit zeitlich gleichbleibendem Zyklus betreiben oder die Saugkurve kann, wie dies im Stand der Technik bekannt ist, in ihrer Form, Frequenz und Intensität, dem Saugverhalten des Säuglings und/oder den Bedürfnissen der Mutter angepasst sein.

Im Deckel 13, d.h. in der Ventilplatte, ist eine erste Auslassöffnung 130 vorhanden, welche die Umwelt mit dem deckelseitigen Teil der Pumpkammer verbindet. Diese Auslassöffnung 130 dient als erster Anschluss für die erste Leitung 2. Eine zweite Auslassöffnung 131, welche ebenfalls den deckel- bzw. brustseitigen Teil der Pumpkammer mit der Umgebung verbindet, ist als zweiter Anschluss ausgebildet. Dieser zweite Anschluss ist mit dem Rückschlagventil 5 versehen. Hier wird ein Schnabelventil verwendet, welches auf einen Stutzen aufgesteckt ist. Andere Ventilarten sind jedoch auch einsetzbar.

Wird die Vorrichtung nun verwendet, so wird die Brusthaube 4 auf die Mutterbrust aufgesetzt, so dass sie mindestens die Brustwarze umfasst. Vorzugsweise ist zusätzlich maximal die Areola von der Brusthaube 4 umfasst. Die Vakuumpumpe 1 wird eingeschaltet und in bekannter Weise betrieben. Das in der Pumpkammer erzeugte Vakuum evakuiert die erste Leitung 2, so dass in der Brusthaube 4 ein Unterdruck vorliegt. Dadurch wird Milch aus der Mutterbrust abgepumpt und gelangt durch die Brusthaube 4 und das Kopplungsteil 3 in die erste Leitung 2. Die Milch fliesst durch den ersten Anschluss 130 in den deckelseitigen Teil der Pumpkammer. Die abgepumpte Milch verlässt die Pumpkammer durch den zweiten Anschluss 131 und das Rückschlagventil 5 und gelangt über die zweite Leitung 6 (siehe Figur 2) bzw. je nach Ausführungsform auch direkt in den Milchsammelbehälter (siehe Figur 6). Es ist somit keine separate Leitung für den Milchtransport vorhanden. Die erste Leitung 2 dient gleichzeitig als Saugleitung und als Milchtransportleitung. Die Vorrichtung wechselt somit nach anfänglichem pneumatischen Pumpen in ein hydraulisches Pumpen. Dies ist eine weitere Annäherung an das natürliche Saugen von Säuglingen.

Die Membran 14 in der Pumpkammer weist drei Funktionen auf. Erstens bildet sie die Membran der Membranvakuumpumpe und erzeugt so das Vakuum in der Pumpkammer. Zweitens dient sie als Scheidewand zwischen der Luft im pumpenseitigen Teil der Pumpkammer und der Milch im deckelseitigen Teil der Pumpkammer. Sie dient somit als Medientrennung. Dadurch verhindert sie, dass Milch in das Pumpaggregat gelangen kann. Sie verhindert aber auch, dass Verschmutzungen des Pumpaggregats in die erste und zweite Leitung 2, 6 gelangen können. Drittens führt ihre zyklische Bewegung innerhalb der Pumpkammer dazu, dass sie die Milch fördert und transportiert. Dank dieser dritten Funktion der Membran 14 können während des Abpumpens Milchsammelbehälter 7, Brusthaube 4 und Vakuumpumpe 1 in voneinander unabhängigen Lagen angeordnet sein. Beispielsweise kann sich der Milchsammelbehälter 7 oberhalb der Vakuumpumpe 1 und/oder der Brusthaube 4 befinden. Auch die Vakuumpumpe 1 kann oberhalb des Milchsammelbehälters 7 und/oder der Brusthaube 4 sein. Dies ermöglicht der Mutter auch liegend abzupumpen oder, wenn sie sitzt, den Milchsammelbehälter 7 und die Vakuumpumpe 1 ausser Reichweite von Kleinkindern auf ein Regal oder eine andere erhöhte Plattform zu stellen.

Vorzugsweise wird in den Beispielen gemäss den Figuren 1 bis 6 ein Unterdruck von 0 bis 300 mmHg erzeugt. Die Pumpfrequenz ist vorzugsweise zwischen 5 und 120 Zyklen pro Minute.

Das Rückschlagventil 5 öffnet sich vorzugsweise erst bei genügendem Druck, d.h. wenn die Pumpkammer ausreichend mit Milch gefüllt ist. Dadurch lässt sich das Totvolumen, welches evakuiert werden muss, minimal halten.

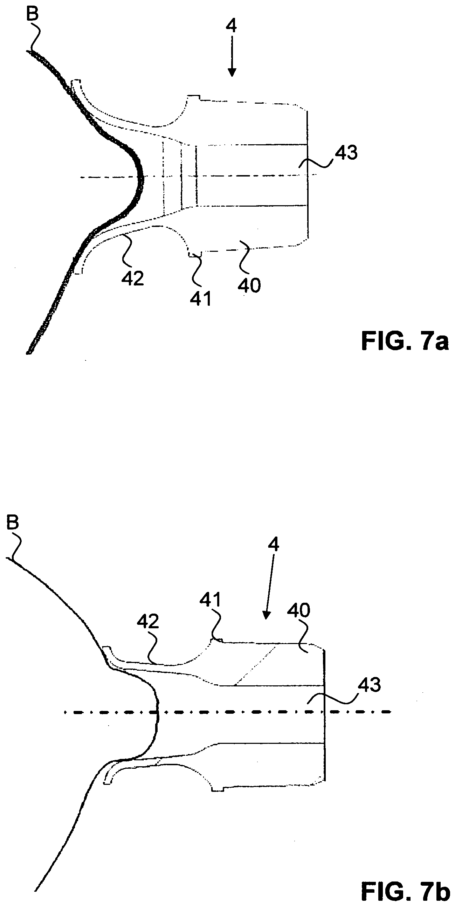

Das Totvolumen lässt sich zudem reduzieren, indem eine kleine Brusthaube 4 verwendet wird, welche nur die Brustwarze und keinen oder einen möglichst geringen Teil der restlichen Brust umfasst. Eine geeignete erfindungsgemässe Brusthaube 4 ist in den Figuren dargestellt. In den Figuren 7 bis 11 ist sie und ihr Kopplungsteil 3 gut erkennbar.

Figur 7a zeigt eine erfindungsgemässe Brusthaube 4, wie sie an eine weibliche Brust B angelegt ist. Die Brusthaube 4 weist einen Stutzen 40, einen Trichter 42 und einen diese zwei verbindenden Übergangsbereich 44 auf. Vorzugsweise ist zwischen Stutzen 40 und Trichter 42 ein radial nach aussen vorstehender Flansch 41 vorhanden. Ein durchgehender Kanal 43 erstreckt sich durch die gesamte Brusthaube 4, so dass diese an zwei gegenüberliegenden Enden offen ausgebildet ist. Vorzugsweise ist die Brusthaube 4 rotationssymmetrisch ausgebildet.

Vorzugsweise ist die Brusthaube 4 einstückig ausgebildet. Sie besteht üblicherweise aus Kunststoff, vorzugsweise aus Silikon.

Der Stutzen 40 ist massiv, d.h. relativ steif ausgebildet und dient zur Kopplung mit dem Kopplungsteil 3. Er weist eine um ein Vielfaches grössere Wandstärke auf als der Trichter 42. In diesem Beispiel ist der Stutzen 40 an seinem äusseren Umfang konisch ausgebildet, wobei er vorzugsweise rotationssymmetrisch ausgebildet ist. Die Konizität erleichtert das Einschieben in das Kopplungsteil 3 und erhöht zudem die Dichtheit der Verbindung mit dem Kopplungsteil 3. Die Dichtheit wird ferner erzielt, indem der Stutzen 40 materialbedingt im Kopplungsteil leicht zusammengedrückt wird. Hierfür ist der Aussendurchmesser des Stutzens 40 leicht grösser als der Innendurchmesser des Kopplungsteils 3. Typische äussere Durchmesser des Stutzens 40 betragen 8 mm bis 40 mm. Typische Längen betragen 5 mm bis 40 mm.

Der Trichter 42 dient zur Aufnahme der Mutterbrust. Er ist vorzugsweise sehr flexibel gestaltet. Er ist wesentlich flexibler und weicher ausgestaltet als der Stutzen 40. Dank seiner Flexibilität passt er sich in seiner Form der Form der Brust an. Er weist hier eine um ein Vielfaches kleinere Wandstärke auf als der Stutzen 40. Alternativ können Stutzen 40 und Trichter 42 auch dieselbe Wandstärke aufweisen, wobei in diesem Fall der Stutzen 40 vorzugsweise aus einem Material mit grösserer Shore-Härte gefertigt ist oder eine Verstärkung aufweist. Vorzugsweise weist der Trichter 42 eine Shore A Härte von circa 50 und der Stutzen 40 eine Shore A Härte von circa 70 auf.

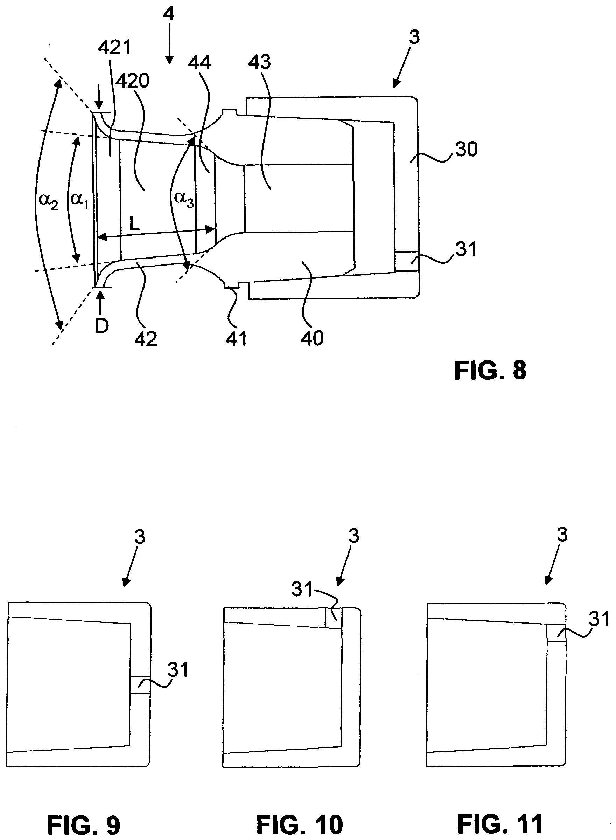

In Figur 8 ist die Brusthaube 4 bei Nichtgebrauch dargestellt. Der Trichter 42 weist mindestens zwei Bereiche auf: einen Hauptbereich 420 und einen vorderen, brusthaubenseitigen Endbereich 421. Der Hauptbereich 420 weist bei Nichtgebrauch einen ersten Öffnungswinkel α₁ und der Endbereich einen zweiten Öffnungswinkel α₂ auf. Der erste Öffnungswinkel α₁ ist kleiner als der zweite Öffnungswinkel α₂. Zudem ist im Endbereich 421 die Wand vorzugsweise nach aussen umgebogen. Wie in den Figuren 7a und 7b erkennbar ist, lässt sich der erste Öffnungswinkel α₁ bei axialem Anpressdruck auf die Brust vergrössern, so dass sich der Endbereich 421 optimal an die Form der Brust anpassen kann.

Wie in den Figuren erkennbar ist, schliesst der Hauptbereich 420 unmittelbar an den Endbereich 421 an. Der Hauptbereich 420 schliesst am anderen Ende unmittelbar an den Übergangsbereich 44 an.

Im Übergangsbereich 44 ist ein weiterer dritter Öffnungswinkel α₃ vorhanden, welcher ebenfalls grösser als der erste Öffnungswinkel α₁ des Hauptbereichs 420 ist. Dieser dient dem Hauptbereich als Vorgabe beim Vergrössern des Winkels.

Die Winkel bei Nichtgebrauch betragen vorzugsweise für den ersten Öffnungswinkel α₁ ≤ 5°, für den zweiten Öffnungswinkel α₂ 90° bis 160° und für den dritten Öffnungswinkel α₃ 60° bis 150°. Mindestens der erste Öffnungswinkel α₁ lässt sich bei Gebrauch vergrössern, vorzugsweise auf einen Winkel von bis zu 10°.

Der Trichter 42 weist eine Länge L von 10 mm bis zu 40 mm auf. Der Durchmesser D im vorderen Endbereich beträgt vorzugsweise 5 mm bis 40 mm, vorzugsweise 20 mm bis 40 mm. Dadurch ist er so klein, dass er die Brustwarze und höchstens noch einen Teil oder die gesamte Areola umfasst. Dies entspricht etwa dem Anteil der Brust, welche von einem Säugling in den Mund genommen wird. Der Trichter 42 ist im Bereich des Stutzens 40 kegelstumpfförmig gestaltet, wobei er sich zur Brust hin öffnet. Sein vorderer, brustseitiger Rand ist stärker nach aussen geneigt als der stutzenseitige Teil.

Da die Brusthaube 4 oder zumindest der Trichter 42 flexibel ausgestaltet ist, kann die Mutter durch Wahl des Anpressdrucks selber wählen, wie viel der Brust tatsächlich von der Brusthaube 4 umfasst wird. Der Anpressdruck ergibt sich aus dem axialen Druck auf den Trichter 42 und dem Gegendruck von der Mutterbrust. In Figur 7a ist der Anpressdruck relativ gross und der Trichter gespreizt, in Figur 7b ist der Druck kleiner und der Trichter 42 umschliesst lediglich die Brustwarze. Durch Wahl des Anpressdrucks lässt sich auch die Dichtheit der Anlage an der Brust einstellen und somit das Abpumpen für die Mutter so bequem wie möglich einstellen.

Die Brusthaube 4 ist, wie dies in Figur 8 erkennbar ist, in das Kopplungsteil 3 eingesteckt. Das Kopplungsteil 3 ist vorzugsweise ebenfalls klein, aber möglichst steif ausgestaltet. Es ist in seinem äusseren Umfang vorzugsweise zylinderförmig ausgebildet und in seinem inneren Umfang kegelstumpfförmig bzw. konisch. Es weist einen u-förmigen Querschnitt auf, ist also an einem Ende offen und am gegenüberliegenden Ende geschlossen ausgebildet. Somit ist ein Sackloch vorhanden, in welches der Stutzen 40 der Brusthaube 4 bis zum Anschlag 41 eingeschoben werden kann. In Figur 8 ist die Brusthaube 4 noch nicht vollständig eingeschoben. Wie jedoch erkennbar ist, ist im vollständig eingeschobenen Zustand zwischen stirnseitigem Ende des Stutzens 40 und der Rückwand des Kopplungsteils 3 ein Spalt vorhanden, welcher einen Fluiddurchlass vom Kanal 43 zu einer Anschlussöffnung 31 im Kopplungsteil 3 bildet.

Diese Anschlussöffnung 31 dient zum Anschluss der ersten Leitung 2. Die erste Leitung 2 kann hier einfach eingesteckt sein, sie kann fest mit dem Kopplungsteil 3 verbunden sein oder es können Ein- oder Aufsteckmittel, beispielsweise Stutzen, zur Verbindung mit der ersten Leitung 2 vorhanden sein.

Die Anschlussöffnung 31 kann an verschiedenen Orten angebracht sein. In Figur 8 ist sie im oberen Bereich in der Rückwand des Kopplungsteils 3 angeordnet. In Figur 9 ist sie mittig in der Rückwand angeordnet. In Figur 10 ist sie im Mantel, jedoch im hinteren, der Rückwand nahen Bereich angeordnet. Vorzugsweise ebenfalls im oberen Bereich. Figur 11 zeigt nochmals die Situation gemäss Figur 8, jedoch ohne eingesteckte Brusthaube 4.

Ist die Anschlussöffnung 31 oben angeordnet, so wird die im Kopplungsteil 3 vorhandene Restluft zusammen mit der Milch abgesaugt und das Totvolumen wird nochmals reduziert. Brusthaube 4 und Kopplungsteil 3 weisen im Gebrauchszustand keine Luftkammern mehr auf. Ihre Freiräume, sofern noch vorhanden, sind mit Milch gefüllt. Damit die Anschlussöffnung 31 bei Gebrauch tatsächlich nach oben gerichtet ist, kann beispielsweise eine entsprechende Markierung auf dem Kopplungsteil 3 angegeben sein.

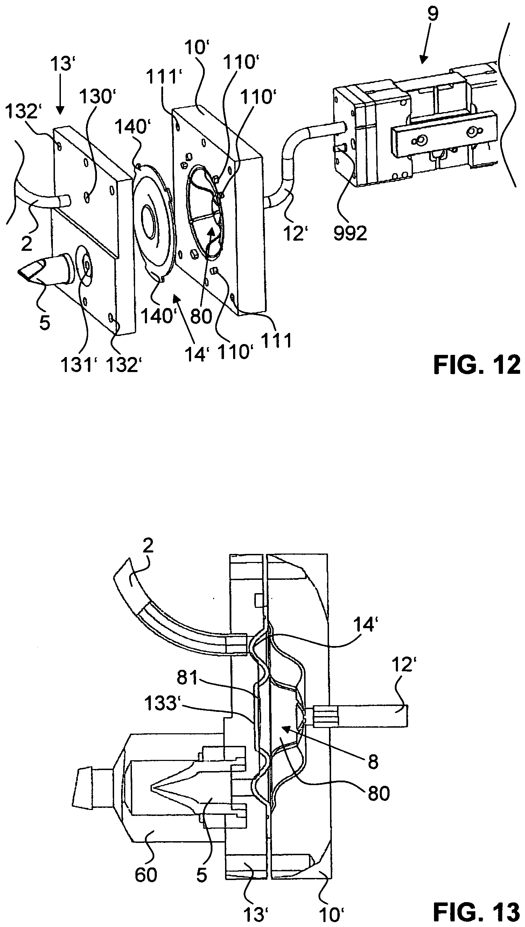

In den Figuren 12 bis 14 ist eine alternative Ausführungsform dargestellt. Die Saugleitung 2 bildet auch hier die Milchleitung. Die abgepumpte Milch wird von der Brusthaube 4 über die erste Leitung brustseitig zu einer Kammer 8 und von dort in den Milchsammelbehälter 7 weiter transportiert. Des Weiteren ist auch hier in der Kammer 8 eine Membran 14' angeordnet, welche die Kammer 8 in einen brusthaubenseitigen und einem antriebsseitigen oder aggregatseitigen Bereich unterteilt.

Diese Membran 14' ist nun jedoch nicht, wie in den vorherigen Beispielen, die Membran der Vakuumpumpe, sondern eine zusätzliche Membran. Sie hat jedoch nach wie vor die Funktion der Medientrennung und dient als Scheidewand zwischen dem im Gebrauch milchgefüllten brusthaubenseitigen Teil 81 und dem einen Unterdruck aufweisenden und mit Luft gefüllten aggregat- oder pumpenseitigen Teil 80. Des Weiteren wird die Membran 14' nach wie vor zyklisch bewegt, wie im Folgenden beschrieben wird. Dadurch transportiert sie die Milch von der Brusthaube 4 durch den brusthaubenseitigen Teil 81 in den Milchsammelbehälter 7, unabhängig von der relativen Lage der Brusthaube 4, der Vakuumpumpe 9 und des Milchsammelbehälters 7 zueinander. Auch hier wechselt das System von einer pneumatischen in eine hydraulische Pumpe.

Die hier dargestellte Membran 14' weist einen kreisförmigen Grundriss auf, wobei sie seitlich vorstehende Flügel 140' aufweist. Hier sind drei Flügel 140' vorhanden. Die Grundplatte 10', welche wiederum Teil eines Vakuumpumpengehäuses sein kann, weist seitliche Anschläge 110' auf, zwischen welchen diese Flügel 140' gehalten sind. Dadurch lässt sich die Membran 14' in eindeutiger Lage in der Kammer 8 halten. Dies erleichtert die Montage. Diese Flügel lassen sich auch in den anhand der Figuren 1 bis 11 beschriebenen Ausführungsformen einsetzen.

Die hier beschriebene Ausführungsform weist eine separate Membranpumpe, hier Pumpenaggregat 9 genannt, auf. Die Membran 14' ist über eine Vakuumleitung 12' mit dem Pumpaggregat 9 verbunden und aufgrund des zyklisch wechselnden Drucks in der Vakuumleitung 12' vom Pumpaggregat 9 angetrieben.

Der Deckel 13' weist zwei Anschlüsse 130' und 131' für die erste und zweite Leitung 2, 6 bzw. für den Milchsammelbehälter 7 auf. Auch hier ist der zweite Anschluss 131' vorzugsweise mit einem Rückschlagventil 5 versehen. Die Verbindung mit der Gegenplatte, welche wiederum Teil eines Pumpengehäuses 10 sein kann, erfolgt vorzugsweise über Schnapp- oder Schraubverbindungen, wobei die entsprechenden Löcher in der Figur 12 mit den Bezugszeichen 132' und 111' versehen sind.

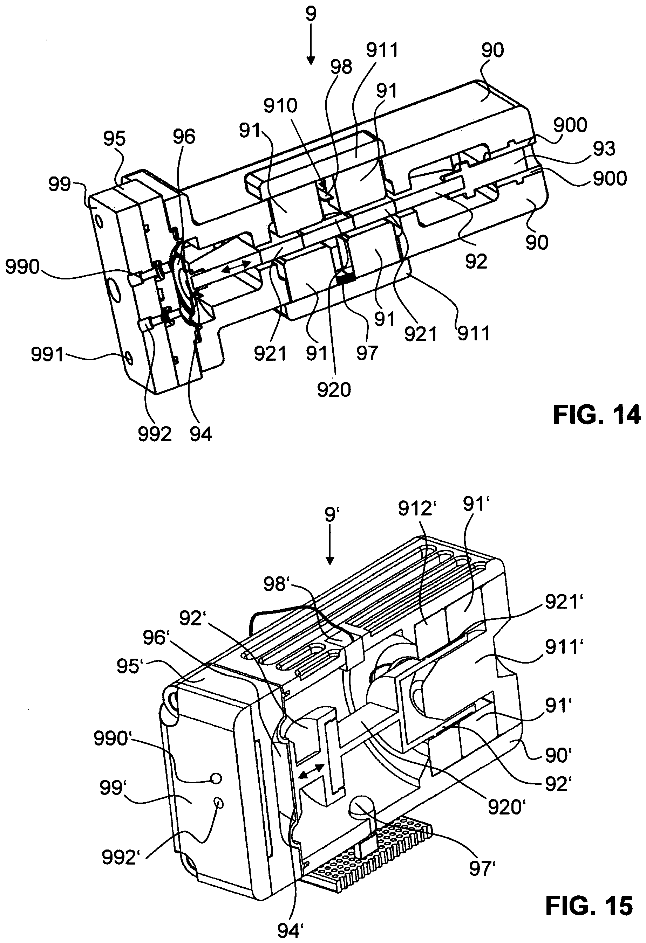

In Figur 14 ist nun das erfindungsgemässe Pumpaggregat 9 gut erkennbar. Es weist ein Gehäuse 90 auf, welches vorzugsweise aus Metall oder Kunststoff gefertigt ist. Das Gehäuse 90 weist vorzugsweise eine quaderförmige Gestalt auf. Im Gehäuse 90 ist auf einer Seite ein flacher Eisenkern 911 und ein daran befestigter Dauermagnet 91 angeordnet. Der Eisenkern 911 liegt auf dem Gehäuse 90 auf, so dass der Dauermagnet 91 in einer Ausnehmung des Gehäuses angeordnet ist. Der Dauermagnet 91 besteht aus zwei Teilen, welche beabstandet zueinander angeordnet sind, so dass zwischen ihnen eine Aussparung 910 vorhanden ist.

Auf einer gegenüberliegenden Seite des Gehäuses ist der identische Aufbau nochmals vorhanden. Auch hier sind ein Eisenkern 911 und ein daran befestigter zweiteiliger Dauermagnet 91 angeordnet.

Zwischen diesen zwei einander gegenüberliegenden Dauermagnetpaaren 91 verläuft ein flacher Spulenkörper 92 mit einer in den Spulenkörper 92 eingelegten Spule 921. Der Spulenkörper 92 ist im Wesentlichen stabförmig oder plattenförmig ausgebildet. An einem Ende ist er in einem Führungslager 93 lagefixiert gehalten. Das Führungslager 93 ist mit dem Spulenkörper 92 inklusive der Spule 921 relativ zum Gehäuse 90 und somit relativ zum Dauermagneten 91 entlang der Längsachse des Gehäuses 90 verschiebbar. Hierfür weist das Gehäuse 90 ein Gleitlager 900 auf. Die Bewegung des Spulenkörpers 92 ist in der Figur 14 mit einem Doppelpfeil dargestellt.

Das andere Ende des Spulenkörpers 92 ist fest mit einer Membran, hier Vakuummembran 94 genannt, verbunden. Die Vakuummembran 94 liegt am stirnseitigen Ende des Gehäuses 90 an diesem an und ist zwischen Gehäuse 90 und einer Ventilplatte 95 festgeklemmt. Die Membran 94 trennt eine Pumpkammer 96 vom Spulenkörper 92. Die Vakuummembran 94 weist vorzugsweise einen kreisförmigen Grundriss auf, wobei sie vorzugsweise eine für Membranen von Membranvakuumpumpen übliche Form aufweist. Die Ventilplatte 95 ist zwischen dem Gehäuse 90 und einem Deckel 99 gehalten. Vorzugsweise sind diese drei Teile lösbar oder unlösbar dicht miteinander verbunden, beispielsweise verschraubt. Die entsprechenden Löcher sind im Deckel 99 mit der Bezugsziffer 991 versehen. Der Deckel 99 weist eine Anschlussöffnung 990 für die Vakuumleitung 12' auf, welche mit der Pumpkammer 96 verbunden ist. Eine Lufteinlassöffnung 992 im Deckel 99 verbindet ebenfalls die Umwelt über die Ventilplatte 95 mit der Pumpkammer 96. Die Ventilplatte 95 weist die für Membranvakuumpumpen üblichen Ventile, Ein- und Auslässe auf. Diese werden hier nicht im Detail beschrieben.

Fliesst ein elektrischer Wechselstrom durch die Spule 921, so verändert sich das elektromagnetische Feld und der Spulenkörper 92 bewegt sich relativ zum Dauermagneten 91. Der Spulenkörper 92 wirkt wie ein Kolben oder Stössel und bewegt die Vakuummembran 94 zyklisch hin und her. Die Kraft, welche auf die Vakuummembran 94 wirkt, ist dabei proportional zu einem an die Spule angelegten Strom. Durch die Bewegung der Vakuummembran 94 wird in der Pumpkammer 96 ein sich zyklisch änderndes Vakuum aufgebaut. Diese Druckänderungen werden über die Vakuumleitung 12' an die Kammer 8 weitergeben, wo die Membran 14' analog bewegt wird.

Die Vakuummembran 94 des Pumpaggregats 9 wird somit über eine elektromagnetisch erzeugte lineare Bewegung angetrieben. Vorteilhaft ist, dass die Bewegung im Vergleich zu den sonst üblichen rotierenden Antrieben ruhiger ist und weniger Vibrationen und Körperschall erzeugt werden. Die Hublänge lässt sich im Gegensatz zum Stand der Technik verändern. Sie kann elektronisch gesteuert werden. Dies erlaubt eine präzise Regelung auch bei tiefen Vakuumpegeln.

Damit die Hublänge gezielt gesteuert werden kann, wird der Weg bzw. die Position des Spulenkörpers 92 überwacht. Hierfür werden Positions- und/oder Bewegungssensoren verwendet. In diesem Beispiel erfolgt dies durch einen optischen Sensor, welcher eine Positionsskala detektiert. Die Positionsskala 920 ist vorzugsweise auf dem Spulenkörper 92 aufgebracht. Eine Lichtquelle 97 sendet ihr Licht senkrecht zur Längsrichtung des Spulenkörpers 92 zu einem gegenüberliegenden Detektor 98, wobei es die Positionsskala 920 passiert. Der Spulenkörper ist in diesem Bereich transparent ausgebildet. Vorzugsweise sind die Lichtquelle 97 und der Detektor 98 in den Ausnehmungen 910 des Dauermagneten 91 angeordnet. Andere Arten der Positionsmessung sind möglich. Das gemessene Signal wird an eine elektronische Steuerung der Vakuumpumpe gesendet und der Strom wird nach Massgabe dieses Signals an die Spule angelegt. Dadurch lassen sich die Position, die Auslenkungsamplitude und die Frequenz unabhängig voneinander steuern. Es werden üblicherweise Vakuumwerte von 0 bis zu 300 mmHg erzielt. Die Frequenzen sind üblicherweise 0 bis 150 Zyklen pro Minute.

Alternativ kann anstelle des Führungslagers 93 auch eine zweite Membran angeordnet sein, welche ähnlich oder identisch zur Vakuummembran 94 ausgebildet ist. Dadurch ist ein symmetrischer Aufbau vorhanden, welcher ebenfalls eine Führung und somit eine lineare Bewegung des Spulenkörpers 92 innerhalb des Gehäuses 90 gewährleistet. Die zweite Membran kann zudem ebenfalls zum Aufbau des Vakuums verwendet werden, so dass die Flussrate gesteigert werden kann.

Figur 15 zeigt eine weitere Ausführungsform einer Vakuumpumpe mit einer elektromagnetisch erzeugten linearen Antriebsbewegung der Membran. Im Gegensatz zur oben beschriebenen Flachspule ist hier eine Tauchspule eingesetzt. Magnet und Spule sind ringförmig bzw. zylinderförmig ausgebildet. Auch hier ist ein Gehäuse 90' vorhanden. Ein Dauermagnet 91' ist am hinteren, der Brusthaube entfernten Ende der Tauchspule angeordnet. Er ist von einem Eisenkern 911' durchsetzt, welcher auf einer ersten Stirnseite des Dauermagneten 91' anliegt. Auf der gegenüberliegenden Stirnseite des Dauermagneten liegt ein Eisenring 912' an. Der Spulenkörper 92' umgreift den Eisenkern 911' und ist durch diesen axial geführt. Der Spulenkörper 92' erstreckt sich zwischen dem Eisenkern 911' und dem Dauermagneten 91' bzw. dem Eisenring 912'. Er ist im Dauermagneten 91' und im Eisenring 912' berührungslos geführt. Die umwickelte Spule 921' umgibt dabei den Spulenkörper 92' in diesem Bereich.

Auch hier ist der Spulenkörper 92' mit der Vakuummembran 94' fest verbunden und wirkt als Kolben für den linearen Antrieb der Vakuummembran 94'. Die Pumpkammer ist mit der Bezugsziffer 96', die Ventilplatte mit der Bezugsziffer 95' und der Deckel mit der Bezugsziffer 99' versehen. Die Anschlussöffnung weist die Bezugsziffer 990' und die Lufteinlassöffnung 992' auf. Auch hier ist wiederum ein Positionssensor vorhanden, welcher die Position des Spulenkörpers 92' relativ zum Permanentmagnet 91' und somit die Bewegung bzw. Position der Vakuummembran 94' an eine Steuerung meldet, um das Vakuum zu regulieren. Die Lichtquelle ist hier mit der Bezugziffer 97', der Detektor mit 98' und die Positionsskala mit 920' bezeichnet. Die Positionsskala ist vorzugsweise transparent ausgeführt. Im Gegensatz zum vorherigen Beispiel ist hier dieser Sensor ausserhalb des Bereichs des Dauermagneten 91' angeordnet.

Die zwei anhand der Figuren 12 bis 15 beschriebenen Vakuumpumpen lassen sich auch in bekannten Brustpumpen verwenden. Das heisst beispielsweise in Systemen, bei welchen die Saugleitung zwischen Vakuumpumpe und Brusthaube getrennt vom Milchfluss erfolgt.

Die Elemente der oben beschriebenen Ausführungsformen lassen sich einzeln oder in Gruppen miteinander kombinieren, um weitere Ausführungsformen zu bilden.

**BEZUGSZEICHENLISTE**

| | | | |
|---|---|---|---|
| 1 | Vakuumpumpe | 5 | Rückschlagventil |
| 10 | Gehäuse | | |
| 11 | Motor | 6 | zweite Leitung |
| 110' | seitlicher Anschlag | 60 | Ventilkappe |
| 111, 111' | Loch | | |
| 112 | Ausnehmung | 7 | Milchsammelbehälter |
| 12 | Kraftübertragungseinheit | 70 | Verbindungsstutzen |
| 12' | Vakuumleitung | | |
| 13, 13' | Deckel | 8 | Pumpkammer |
| 130, 130' | erster Anschluss | 80 | antriebsseitiger Kammerteil |
| 131, 131' | zweiter Anschluss | 81 | brusthaubenseitiger Kammerteil |
| 132, 132' | Loch | | |
| 133, 133' | Ausnehmung | | |
| 14, 14' | Membran | 9, 9' | Pumpaggregat |
| 140' | seitlicher Flügel | 90, 90' | Gehäuse |
| | | 900 | Gleitlager |
| 2 | erste Leitung | 91, 91' | Dauermagnet |
| | | 910 | Ausnehmung |
| 3 | Kopplungsteil | 911, 911' | Eisenkern |
| 30 | Grundkörper | 912' | Eisenring |
| 31 | Anschlussöffnung | 92, 92' | Spulenkörper mit Spule |
| | | 920, 920' | Positionsskala |
| 4 | Brusthaube | 921, 921' | Spule |
| 40 | Stutzen | 93 | Führungslager |
| 41 | Anschlag | 94, 94' | Vakuummembran |
| 42 | Trichter | 95, 95' | Ventilplatte |
| 420 | Hauptbereich | 96, 96' | Pumpkammer |
| 421 | Übergangsbereich | 97, 97' | Lichtquelle |
| 43 | Kanal | 98, 98' | Detektor |
| 44 | Übergangsbereich | 99, 99' | Deckel |
| | | 990 | Anschlussöffnung |
| 991 | Loch | L | Länge des Trichters |
| 992 | Lufteinlassöffnung | α₁ | erster Öffnungswinkel |
| | | α₂ | zweiter Öffnungswinkel |
| B | Brust | α₃ | dritter Öffnungswinkel |
| D | Durchmesser des Endbereichs des Trichters | | |

## Patentansprüche

1. Vorrichtung zum Abpumpen von menschlicher Muttermilch, wobei die Vorrichtung eine Brusthaube (4) zur Anlage an eine Mutterbrust, eine Vakuumpumpe (1, 9, 9') zur Erzeugung eines Vakuums, eine die Vakuumpumpe (1, 9, 9') mit der Brusthaube (4) verbindenden Leitung (2) zur Übermittlung des erzeugten Vakuums an die Brusthaube (4), eine Kammer (133, 112; 8) und einen Milchsammelbehälter (7) aufweist, wobei die Leitung (2) pumpenseitig in einem ersten Anschluss (130, 130') dieser Kammer (133, 112; 8) endet, **dadurch gekennzeichnet, dass** die Kammer (133, 112; 8) einen zweiten Anschluss (131, 131') aufweist, über welchen sie mit dem Milchsammelbehälter (7) verbunden ist, dass die zwei Anschlüsse (130, 130', 131, 131') in der Kammer (133, 112; 8) in fluidkommunizierender Verbindung miteinander stehen und dass die Leitung (2) während des Abpumpens eine Milchleitung bildet zum Transport von in der Brusthaube (4) abgepumpter Muttermilch zur Kammer (133, 112; 8) und von der Kammer (133, 112; 8) zum Milchsammelbehälter (7).

2. Vorrichtung nach Anspruch 1, wobei eine Scheidewand (14, 14') vorhanden ist, welche die Kammer (133, 112; 8) in einen antriebsseitigen Bereich (112, 80) und einen brusthaubenseitigen Bereich (133, 81) trennt und wobei der erste und der zweite Anschluss (130, 130', 131, 131') im brusthaubenseitigen Bereich (133, 81) angeordnet sind.

3. Vorrichtung nach Anspruch 2, wobei die Scheidewand (14, 14') eine Membran ist, wobei die Membran (14, 14') angetrieben ist und zur Förderung der abgepumpten Milch ausgebildet ist.

4. Vorrichtung nach Anspruch 3, wobei die Vakuumpumpe (1) eine Membranpumpe ist, die Kammer (133, 112) eine Pumpkammer der Vakuumpumpe (1) ist und die Membran (14') die Kammer (133, 112) in die zwei genannten Bereiche unterteilt.

5. Vorrichtung nach einem der Ansprüche 3 und 4, wobei der Antrieb der Membran (14) gleichzeitig zur Erzeugung des Vakuums in der Pumpkammer (133, 112) und zur Förderung des Milchflusses dient.

6. Vorrichtung nach einem der Ansprüche 3 bis 5, wobei die Membran (14, 14') einen im Wesentlichen kreisförmigen Grundriss aufweist.

7. Vorrichtung nach einem der Ansprüche 3 bis 6, wobei die Membran (14, 14') in ihrem mittleren Bereich, vorzugsweise ihrer Mitte, angetrieben ist.

8. Vorrichtung nach einem der Ansprüche 1 bis 7, wobei der zweite Anschluss (131, 131') mit einem Einwegventil (5) versehen ist, welches einen Rückfluss von abgesaugter Milch vom Milchsammelbehälter (7) zur Kammer (133, 112; 8) verhindert.

9. Vorrichtung nach einem der Ansprüche 1 bis 8, wobei die Brusthaube (4) einen rohrförmigen Stutzen (40) und einen daran einstückig angeformten Trichter (42) zur Auflage an eine Mutterbrust aufweist, wobei der Trichter (42) sich zu seinem freien, dem Stutzen (40) abgewandten Seite hin erweitert und wobei ein Kanal (43) vorhanden ist, welcher sich von einem brustseitigen Ende des Trichters (42) durchgehend bis zu einem, diesem Ende gegenüberliegenden, pumpenseitigen Ende des Stutzens (40) erstreckt und welcher zum Anlegen eines Vakuums an die Mutterbrust und zum Abfliessen der abgepumpten Muttermilch dient, wobei der Trichter (42) flexibler ausgebildet ist als der Stutzen (40), wobei der Trichter (42) einen sich über einen wesentlichen Teil seiner Länge erstreckenden Hauptbereich (420) mit einem ersten Öffnungswinkel (α₁) des Kanals (43) und einen brustseitigen Endbereich (421) mit einem zweiten Öffnungswinkel (α₂) des Kanals (43) aufweist und wobei der erste Öffnungswinkel (α₁) bei Nichtgebrauch kleiner ist als der zweite Öffnungswinkel (α₂) und dass im Gebrauchszustand mindestens der erste Öffnungswinkel (α₁) durch axialen Druck auf die Brusthaube (4) vergrösserbar ist.

10. Vorrichtung nach einem der Ansprüche 1 bis 9, wobei die Brusthaube (4) einen rohrförmigen Stutzen (40) und einen daran einstückig angeformten Trichter (42) zur Auflage an eine Mutterbrust aufweist, wobei der Trichter (42) einen brustseitigen Durchmesser (D) von 5 mm bis 40 mm und eine Länge (L) von 10 mm bis 40 mm aufweist, so dass im Gebrauchszustand die Brustwarze und maximal die Areola von der Brusthaube (4) umfasst ist.

11. Vorrichtung nach einem der Ansprüche 1 bis 10, wobei die Brusthaube (4) in einem Kopplungsteil (3) angeordnet ist, welcher in Gebrauchslage im oberen Bereich einen Anschluss (31) zum Anschliessen der Leitung (2) aufweist.

12. Vorrichtung nach einem der Ansprüche 1 bis 11, wobei der Milchsammelbehälter (7) direkt am zweiten Anschluss (131, 131') der Kammer (133, 112; 8) befestigt ist oder über eine zweite Leitung (6) mit der Kammer (133, 112; 8) verbunden ist.

13. Verfahren zum Betreiben einer Vorrichtung zum Abpumpen von menschlicher Muttermilch, wobei mittels einer Vakuumpumpe (1, 9, 9') ein Vakuum in einer Brusthaube (4) erzeugt wird, wobei das erzeugte Vakuum von einer Kammer (133, 112; 8), die einen ersten Anschluss (130, 130') hat, über eine Leitung (2), die an dem ersten Anschluss (130, 130') endet, zur Brusthaube (4) geleitet wird und wobei abgepumpte Milch über die Brusthaube (4) in einem Milchsammelbehälter (7) gesammelt wird, **dadurch gekennzeichnet, dass** dieselbe Leitung (2), welche für die Übermittlung des Vakuums verwendet wird, während dem Abpumpen die in der Brusthaube (4) abgepumpte Milch zur Kammer (133, 112; 8) leitet und dass die Milch während dem Abpumpen von der Kammer (133, 112; 8) durch einen an der Kammer angeordneten zweiten Anschluss (131, 131') in den Milchsammelbehälter (7) weitergeleitet wird, und dass die Kammer (133, 112; 8) durch eine Membran (14, 14') in einen antriebsseitigen Bereich (112, 80) und einen brusthaubenseitigen Bereich (133, 81) getrennt wird, der erste und der zweite Anschluss (130, 130', 131, 131') im brusthaubenseitigen Bereich (133, 81) angeordnet sind und die zur Förderung der abgepumpten Milch ausgebildete Membran (14, 14') angetrieben wird.

14. Verfahren nach Anspruch 13, wobei ein Rückschlagventil (5) verwendet wird, welches bei genügendem Druck in der Kammer (133, 112; 8) eine Verbindung zwischen der Kammer (133, 112; 8) und dem Milchsammelbehälter (7) schafft.

## Claims

1. A device for expressing human breast milk, wherein the device has a breast shield (4) for bearing against a mother's breast, a vacuum pump (1, 9, 9') for generating a vacuum, a line (2) which connects the vacuum pump (1, 9, 9') to the breast shield (4) and is intended for transmitting the vacuum generated to the breast shield (4), a chamber (133, 112; 8) and a milk collecting container (7), the line (2) ending on the pump side in a first port (130, 130') of said chamber (133, 112; 8), **characterized in that** the chamber (133, 112; 8) has a second port (131, 131'), through which it is connected to the milk collecting container (7), **in that** the two ports (130, 130', 131, 131') in the chamber (133, 112; 8) are connected to each other in terms of fluid communication, and **in that**, during the expressing operation, the line (2) forms a milk line for transporting breast milk expressed in the breast shield (4) to the chamber (133, 112; 8) and from the chamber (133, 112; 8) to the milk collecting container (7).

2. The device as claimed in claim 1, wherein there is a partition wall (14, 14'), which separates the chamber (133, 112; 8) into a drive-side region (112, 80) and a breast-shield-side region (133, 81), and wherein the first and the second port (130, 130', 131, 131') are arranged in the breast-shield-side region (133, 81).

3. The device as claimed in claim 2, wherein the partition wall (14, 14') is a diaphragm, wherein the diaphragm (14, 14') is driven and is designed for conveying the expressed milk.

4. The device as claimed in claim 3, wherein the vacuum pump (1) is a diaphragm pump, the chamber (133, 112) is a pump chamber of the vacuum pump (1), and the diaphragm (14') divides the chamber (133, 112) into the two regions mentioned.

5. The device as claimed in claim 3 or 4, wherein the drive of the diaphragm (14) serves simultaneously to generate the vacuum in the pump chamber (133, 112) and to convey the flow of milk.

6. The device as claimed in one of claims 3 to 5, wherein the diaphragm (14, 14') has a substantially circular outline.

7. The device as claimed in one of claims 3 to 6, wherein the diaphragm (14, 14') is driven in the central region thereof, preferably in the center thereof.

8. The device as claimed in one of claims 1 to 7, wherein the second port (131, 131') is provided with a one-way valve (5), which prevents sucked off milk from flowing back from the milk collecting container (7) to the chamber (133, 112; 8).

9. The device as claimed in one of claims 1 to 8, wherein the breast shield (4) has a tubular connector (40) and a funnel (42), which is integrally formed thereon and is intended for resting on a mother's breast, wherein the funnel (42) widens toward the free side thereof that faces away from the connector (40), and wherein there is a passage (43), which extends continuously from a breast-side end of the funnel (42) as far as a pump-side end, being opposite said breast-side end, of the connector (40) and which serves to apply a vacuum to the mother's breast and for the flowing away of the expressed breast milk, wherein the funnel (42) is of more flexible design than the connector (40), wherein the funnel (42) has a main region (420) extending over a substantial part of the length thereof with a first opening angle (α₁) of the passage (43), and a breast-side end region (421) with a second opening angle (α₂) of the passage (43), and wherein, when not in use, the first opening angle (α₁) is smaller than the second opening angle (α₂), and wherein, in the use state, at least the first opening angle (α₁) can be enlarged by axial pressure on the breast shield (4).

10. The device as claimed in one of claims 1 to 9, wherein the breast shield (4) has a tubular connecter (40) and a funnel (42) that is integrally formed thereon and is intended for resting against a mother's breast, wherein the funnel (42) has a breast-side diameter (D) of 5 mm to 40 mm and a length (L) of 10 mm to 40 mm, such that, in the use state, the nipple, and at maximum the areola, is surrounded by the breast shield (4).

11. The device as claimed in one of claims 1 to 10, wherein the breast shield (4) is arranged in a coupling part (3), the upper region of which, in the use position, has a port (31) for the connection of the line (2).

12. The device as claimed in one of claims 1 to 11, wherein the milk collecting container (7) is fastened directly to the second port (131, 131') of the chamber (133, 112; 8) or it is connected to the chamber (133. 112; 8) via a second line (6).

13. A method for operating a device for expressing human breast milk, a vacuum being generated in a breast shield (4) by means of a vacuum pump (1, 9, 9'), the vacuum generated being conducted from a chamber (133, 112; 8) having a first port (130, 130') via a line (2) to the breast shield (4), the line (2) ending at the first port (130, 130'), and expressed milk being collected via the breast shield (4) in a milk collecting container (7), **characterized in that**, during the expressing operation, the same line (2), which is used for transmitting the vacuum, conducts the milk expressed in the breast shield (4) to the chamber (133, 112; 8), and **in that**, during the expressing operation, the milk is conducted further from the chamber (133, 112; 8) through a second port (131, 131') arranged at the chamber into the milk collecting container (7) and **in that** the chamber (133, 112; 8) is separated by a diaphragm (14, 14') into a drive-side region (112, 80) and a breast-shield-side region (133, 81), the first and the second port (130, 130', 131, 131') being arranged in the breast-shield-side region (133, 81) and the diaphragm (14, 14') being designed for conveying the expressed milk.

14. The method as claimed in claim 13, wherein use is made of a nonreturn valve (5), which, when there is sufficient pressure in the chamber (133, 112; 8), creates a connection between the chamber (133, 112; 8) and the milk collecting container (7).

## Revendications

1. Dispositif destiné à pomper le lait maternel humain, le dispositif présentant une téterelle (4) destinée à s'appliquer contre le sein d'une mère, une pompe à vide (1, 9, 9') pour produire un vide, une conduite (2) reliant la pompe à vide (1, 9, 9') à la téterelle (4), pour transmettre le vide produit à la téterelle (4), une chambre (133, 112 ; 8) et un récipient de collecte de lait (7), la conduite (2) se terminant du côté de la pompe par un premier raccord (130, 130') de cette chambre (133, 112 ; 8), **caractérisé en ce que** la chambre (133, 112 ; 8) présente un deuxième raccord (131, 131') par le biais duquel elle est connectée au récipient de collecte de lait (7), **en ce que** les deux raccords (130, 130', 131, 131') dans la chambre (133, 112 ; 8) sont en liaison fluidique l'un avec l'autre et **en ce que** la conduite (2), pendant le pompage, forme une conduite de lait pour le transport du lait maternel pompé dans la téterelle (4) vers la chambre (133, 112 ; 8) et depuis la chambre (133, 112 ; 8) vers le récipient de collecte de lait (7).

2. Dispositif selon la revendication 1, dans lequel une paroi de séparation (14, 14') est prévue, laquelle sépare la chambre (133, 112 ; 8) en une région du côté de l'entraînement (112, 80) et une région du côté de la téterelle (133, 81) et dans lequel le premier et le deuxième raccord (130, 130', 131, 131') sont disposés dans la région du côté de la téterelle (133, 81).

3. Dispositif selon la revendication 2, dans lequel la paroi de séparation (14, 14') est une membrane, la membrane (14, 14') étant entraînée et étant réalisée pour refouler le lait pompé.

4. Dispositif selon la revendication 3, dans lequel la pompe à vide (1) est une pompe à membrane, la chambre (133, 112) est une chambre de pompe de la pompe à vide (1) et la membrane (14') divise la chambre (133, 112) en les deux régions mentionnées.

5. Dispositif selon l'une quelconque des revendications 3 et 4, dans lequel l'entraînement de la membrane (14) sert simultanément à produire le vide dans la chambre de pompe (133, 112) et à refouler le flux de lait.

6. Dispositif selon l'une quelconque des revendications 3 à 5, dans lequel la membrane (14, 14') présente un pourtour essentiellement circulaire.

7. Dispositif selon l'une quelconque des revendications 3 à 6, dans lequel la membrane (14, 14') est entraînée dans sa région centrale, de préférence.

8. Dispositif selon l'une quelconque des revendications 1 à 7, dans lequel le deuxième raccord (131, 131') est pourvu d'une soupape unidirectionnelle (5) qui empêche un reflux du lait aspiré depuis le récipient de collecte de lait (7) vers la chambre (133, 112 ; 8).

9. Dispositif selon l'une quelconque des revendications 1 à 8, dans lequel la téterelle (4) présente un embout de forme tubulaire (40) et un entonnoir (42) formé sur celui-ci d'une seule pièce pour l'application contre un sein maternel, l'entonnoir (42) s'élargissant vers son côté libre opposé à l'embout (40) et un canal (43) étant prévu, lequel s'étend depuis une extrémité de l'entonnoir (42) du côté du sein en continu jusqu'à une extrémité de l'embout (40) opposée à cette extrémité, du côté de la pompe, et lequel sert à appliquer un vide au sein maternel et à faire s'écouler le lait maternel pompé, l'entonnoir (42) étant réalisé sous forme plus flexible que l'embout (40), l'entonnoir (42) présentant une région principale (420) s'étendant sur une majeure partie de sa longueur avec un premier angle d'ouverture (α1) du canal (43) et une région d'extrémité (421) du côté du sein avec un deuxième angle d'ouverture (α2) du canal (43) et le premier angle d'ouverture (α1), lors de la non utilisation, étant inférieur au deuxième angle d'ouverture (α2) et en ce qu'à l'état d'utilisation, au moins le premier angle d'ouverture (α1) peut être augmenté par pression axiale sur la téterelle (4).

10. Dispositif selon l'une quelconque des revendications 1 à 9, dans lequel la téterelle (4) présente un embout de forme tubulaire (40) et un entonnoir (42) formé d'une seule pièce sur celui-ci, destiné à être appliqué contre un sein maternel, l'entonnoir (42) présentant un diamètre (D) du côté du sein, de 5 mm à 40 mm et une longueur (L) de 10 mm à 40 mm de sorte qu'à l'état d'utilisation, le mamelon et au maximum l'aréole soient enveloppés par la téterelle (4).

11. Dispositif selon l'une quelconque des revendications 1 à 10, dans lequel la téterelle (4) est disposée dans une partie d'accouplement (3) qui, dans la position d'utilisation, présente dans la région supérieure un raccord (31) pour le raccordement de la conduite (2).

12. Dispositif selon l'une quelconque des revendications 1 à 11, dans lequel le récipient de collecte de lait (7) est fixé directement au deuxième raccord (131, 131') de la chambre (133, 112 ; 8) ou est connecté à la chambre (133, 112 ; 8) par le biais d'une deuxième conduite (6).

13. Procédé pour faire fonctionner un dispositif de pompage de lait maternel humain, un vide étant généré dans une téterelle (4) au moyen d'une pompe à vide (1, 9, 9'), le vide produit étant conduit à la téterelle (4) depuis une chambre (133, 112 ; 8) qui présente un premier raccord (130, 130') par le biais d'une conduite (2) qui se termine au niveau du premier raccord (130, 130'), et le lait pompé par le biais de la téterelle (4) étant recueilli dans un récipient de collecte de lait (7), **caractérisé en ce que** la même conduite (2) qui est utilisée pour le transfert du vide conduit à la chambre (133, 112 ; 8) pendant le pompage le lait pompé dans la téterelle (4) et **en ce que** le lait, pendant le pompage depuis la chambre (133, 112 ; 8), est transféré par un deuxième raccord (131, 131') disposé au niveau de la chambre dans le récipient de collecte de lait (7), et **en ce que** la chambre (133, 112 ; 8) est séparée par une membrane (14, 14') en une région côté entraînement (112, 80) et une région côté téterelle (133, 81), le premier et le deuxième raccord (130, 130', 131, 131') étant disposés dans la région côté téterelle (133, 81) et la membrane (14, 14') réalisée pour le transport du lait pompé étant entraînée.

14. Procédé selon la revendication 13, dans lequel un clapet antiretour (5) est utilisé, lequel, en cas de pression suffisante dans la chambre (133, 112 ; 8), établit une liaison entre la chambre (133, 112 ; 8) et le récipient de collecte de lait (7).
